# EUROPÄISCHE PATENTANMELDUNG

(11) **EP 1 057 469 A2**
(43) Veröffentlichungstag der Anmeldung: **06.12.2000**
(21) Anmeldenummer: 00108423.5
(22) Anmeldetag: 18.04.2000
(51) Int. Cl.: A61K 7/09

(54) **Verfahren zur dauerhaften Verformung von Keratinfasern**

(30) Priorität: 27.04.1999 DE 19919087
(71) Anmelder: Cognis Deutschland GmbH, 40589 Düsseldorf (DE)
(72) Erfinder: Seipel, Werner, 40723 Hilden (DE); Kleen, Astrid, Dr., 40699 Erkrath (DE)

(57) **Zusammenfassung**

Vorgeschlagen wird ein Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült, welches sich dadurch auszeichnet, daß die wäßrige Zubereitung der keratinreduzierenden Substanz und/oder des Oxidationsmittels
(a) 0,1 bis 20 Gew.-% Zuckertenside ausgewählt aus der Gruppe, die gebildet wird von (a1) Alkyl- und/oder Alkenyloligoglykosiden und/oder (a2) Fettsäure-N-alkylpolyhydroxyalkylamiden und
(b) 0,1 bis 20 Gew.-% Fettsäurepartialglyceride
enthält, mit der Maßgabe, daß sich die Mengenangaben mit Wasser sowie gegebenenfalls weiteren Hilfs- und Zusatzstoffen zu 100 Gew.-% ergänzen.

## Beschreibung

### Gebiet der Erfindung

Die Erfindung betrifft ein Verfahren zur dauerhaften Verformung von Keratinfasern unter Einsatz von Zuckertensiden und Partialglyceriden sowie deren Verwendung zur Herstellung von Wellmitteln.

### Stand der Technik

Die dauerhafte Verformung von Keratinfasern wird üblicherweise so durchgeführt, daß man die Faser mechanisch verformt und die Verformung durch geeignete Hilfsmittel festlegt. Vor und/oder nach dieser Verformung behandelt man die Faser mit der wäßrigen Zubereitung einer keratinreduzierenden Substanz und spült nach einer Einwirkungszeit mit Wasser oder einer wäßrigen Lösung. In einem zweiten Schritt behandelt man dann die Faser mit der wäßrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wäßrige Zubereitung des Keratinreduktionsmittels ist üblicherweise alkalisch eingestellt, damit die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so daß es zu einer Lockerung der Peptidvernetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert.

Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Wenngleich dieses als Dauerwelle bezeichnete Verfahren heute in großem Umfang angewendet wird, werden dafür nach wie vor Mittel eingesetzt, die hinsichtlich einer Reihe von Punkten nicht als optimal angesehen werden können. Insbesondere ist man bestrebt, unter Beibehaltung der gewünschten Umformleistung die bei strapaziertem, insbesondere bei oxidativ vorbehandeltem, Haar auftretenden Schädigungen, die bis hin zum Haarbruch gehen können, zu verringern und das Haar vor überhöhter Austrocknung und Feuchtigkeitsverlust zu schützen. Gleiches gilt für die in manchen Fällen auftretenden Probleme im Kopfhautbereich aufgrund von dermatologischer Unverträglichkeit. Ein weiteres Problem besteht ferner darin, daß die Zubereitungen insbesondere bei Temperaturlagerung leicht eindicken und dann nicht mehr problemlos anwendbar sind.

Die Aufgabe der vorliegenden Erfindung hat daher darin bestanden, ein Verfahren zur dauerhaften Verformung von Keratinfasern, insbesondere von menschlichem Haar, zur Verfügung zu stellen, welches frei von den oben geschilderten Nachteilen ist.

### Beschreibung der Erfindung

Gegenstand der Erfindung ist ein Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült, welches sich dadurch auszeichnet, daß die wäßrige Zubereitung der keratinreduzierenden Substanz und/oder des Oxidationsmittels
(a) 0,1 bis 20 Gew.-% Zuckertenside ausgewählt aus der Gruppe, die gebildet wird von (a1) Alkyl- und/oder Alkenyloligoglykosiden und/oder (a2) Fettsäure-N-alkylpoly-hydroxyalkylamiden und
(b) 0,1 bis 20 Gew.-% Fettsäurepartialglyceride
enthält, mit der Maßgabe, daß sich die Mengenangaben mit Wasser sowie gegebenenfalls weiteren Hilfs- und Zusatzstoffen zu 100 Gew.-% ergänzen.

Überraschenderweise wurde gefunden, daß eine wesentliche Verringerung der Schädigung des Haares unter Erhalt oder sogar Steigerung der Umformleistung erzielt werden kann, wenn die eingesetzten wäßrigen Zubereitungen eine Mischung aus (a) nichtionischen Tensiden vom Typ der Alkyl- und/oder Alkenyloligoglykoside und/oder der Fettsäure-N-alkylpoly-hydroxyalkylamide und (b) Fettsäurepartialglyceride enthalten. Diese Mischungen führen nach der Verformung zu einer Stabilisierung und Kräftigung der Haare und liefern somit einen gleichzeitigen Schutz der Haare vor Austrocknung und Feuchtigkeitsverlust. Weiterhin zeigen die Zubereitungen darüber hinaus eine gute dermatologische Verträglichkeit. Die Erfindung schließt die Erkenntnis mit ein, daß sich die Performance weiter verbessern läßt, wenn man zusätzlich Esterquats und/oder Proteinhydrolysate einsetzt.

### Alkyl- und/oder Alkenyloligoglykoside

Alkyl- und/oder Alkenyloligoglykoside, welche die Zuckertensidkomponente (a1) bilden, steilen bekannte nichtionische Tenside dar, die der Formel **(I)** folgen, 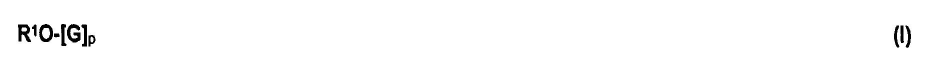 in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in **Starch/Stärke 45, 281 (1993),** B.Salka in **Cosm.Toil. 108, 89 (1993)** sowie J.Kahre et al. in **SÖFW-Journal Heft 8, 598 (1995)** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Im Sinne des erfindungsgemäßen Verfahrens können die Alkyl- und/oder Alkenyloligoglykoside in Mengen von 0,1 bis 20, vorzugsweise 0,5 bis 10 und insbesondere 1 bis 5 Gew.-% - jeweils bezogen auf die keratinreduzierende Substanz bzw. das Oxidationsmittel - eingesetzt werden.

### Fettsäure-N-alkylpolyhydroxyalkylamide

Fettsäure-N-alkylpolyhydroxyalkylamide, welche die Zuckertensidkomponente (a2) bilden, stellen nichtionische Tenside dar, die der Formel **(II)** folgen, 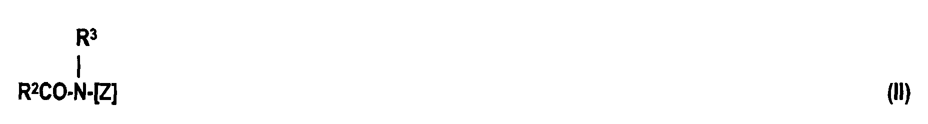 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens. Surf. Det. 25, 8 (1988)**. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(III)** wiedergegeben werden:

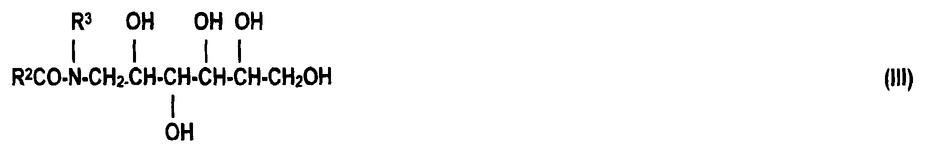

Vorzugsweise werden als Fettsäure-N-alkylpolyhydroxyalkylamide Glucamide der Formel **(III)** eingesetzt, in der R³ für Wasserstoff oder eine Alkylgruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(III)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

Im Sinne des erfindungsgemäßen Verfahrens können die Fettsäure-N-alkylpolyhydroxyalkylamide in Mengen von 0,1 bis 20, vorzugsweise 0,5 bis 10 und insbesondere 1 bis 5 Gew.-% - jeweils bezogen auf die keratinreduzierende Substanz bzw. das Oxidationsmittel - eingesetzt werden.

### Fettsäurepartialglyceride

Fettsäurepartialglyceriden, welche die Komponente (b) bilden, also Monoglyceride, Diglyceride und deren technische Gemische können herstellungsbedingt noch geringe Mengen Triglyceride enthalten. Die Partialglyceride folgen vorzugsweise der Formel **(IV)**, 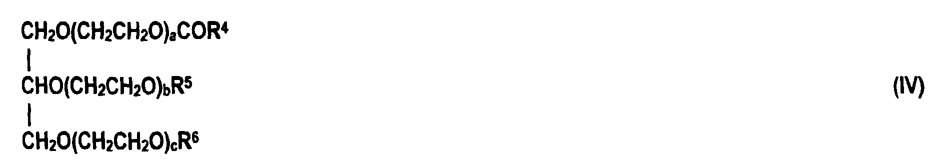 in der R⁴CO für einen linearen oder verzweigten, gesättigten und/oder ungesättigten Acylrest mit 6 bis 22, vorzugsweise 12 bis 18 Kohlenstoffatomen, R⁵ und R⁶ unabhängig voneinander für R⁴CO oder OH und die Summe (a+b+c) für 0 oder Zahlen von 1 bis 100, vorzugsweise 5 bis 25 steht, mit der Maßgabe, daß mindestens einer der beiden Reste R⁵ und R⁶ OH bedeutet. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Ölsäuremonoglyceride. Vorzugsweise werden Ölsäure monoglyceride eingesetzt.

Im Sinne des erfindungsgemäßen Verfahrens können die Fettsäurepartialglyceride in Mengen von 0,1 bis 20, vorzugsweise 0,5 bis 10 und insbesondere 1 bis 5 Gew.-% - jeweils bezogen auf die keratinreduzierende Substanz bzw. das Oxidationsmittel - eingesetzt werden.

### Esterquats

Unter der Bezeichnung 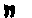 Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE 4308794 C1** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Übersichten zu diesem Thema sind beispielsweise von R. Puchta et al. in **Tens.Surf.Det., 30, 186 (1993),** M. Brock in **Tens.Surf.Det. 30, 394 (1993),** R. Lagerman et al. in **J.Am.Oil.Chem.Soc., 71, 97 (1994)** sowie I. Shapiro in **Cosm.Toil. 109, 77 (1994)** erschienen.

Die quaternierten Fettsäuretriethanolaminestersalze folgen der Formel **(V),** 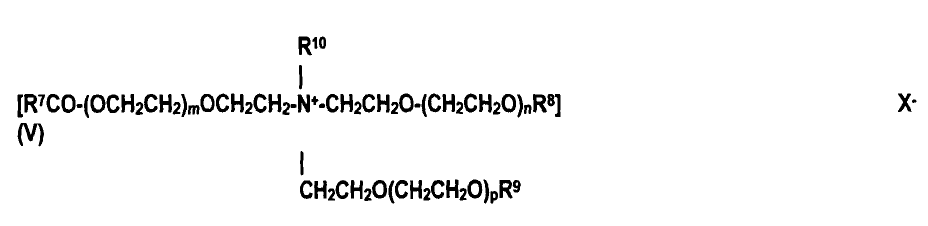 in der R⁷CO für einen Acylrest mit 6 bis 22 Kohlenstoffatomen, R⁸ und R⁹ unabhängig voneinander für Wasserstoff oder R⁷CO, R¹⁰ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (Iodzahl 0 bis 40) ab. Aus anwendungstechnischer Sicht haben sich quaternierte Fettsäuretriethanolaminestersalze der Formel **(V)** als besonders vorteilhaft erwiesen, in der R⁷CO für einen Acylrest mit 16 bis 18 Kohlenstoffatomen, R⁸ für R⁷CO, R⁹ für Wasserstoff, R¹⁰ für eine Methylgruppe, m, n und p für 0 und X für Methylsulfat steht.

Neben den quaternierten Fettsäuretriethanolaminestersalzen kommen als Esterquats ferner auch quaternierte Estersalze von Fettsäuren mit Diethanolalkylaminen der Formel **(VI)** in Betracht, 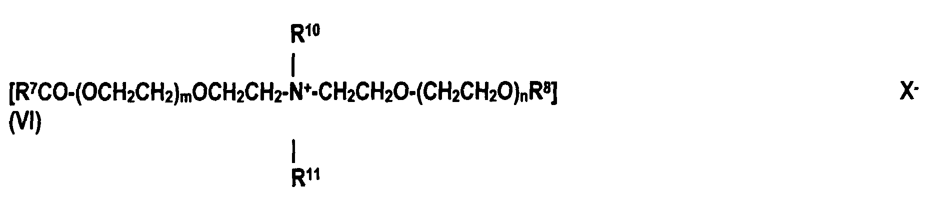 in der R⁷CO für einen Acylrest mit 6 bis 22 Kohlenstoffatomen, R⁸ für Wasserstoff oder R⁷CO, R¹⁰ und R¹¹ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(VII)** zu nennen, 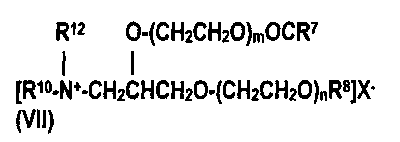 in der R⁷CO für einen Acylrest mit 6 bis 22 Kohlenstoffatomen, R⁸ für Wasserstoff oder R⁷CO, R¹⁰, R¹² und R¹³ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Schließlich kommen als Esterquats noch Stoffe in Frage, bei denen die Ester- durch eine Amidbindung ersetzt ist und die vorzugsweise basierend auf Diethylentriamin der Formel **(VIII)** folgen, 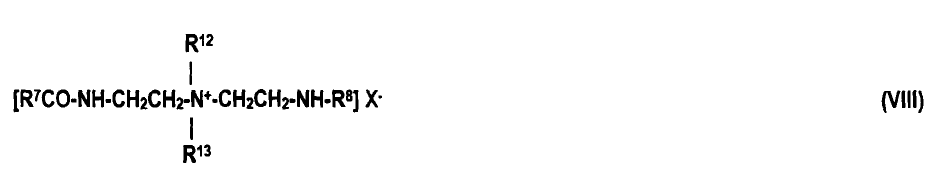 in der R⁷CO für einen Acylrest mit 6 bis 22 Kohlenstoffatomen, R⁸ für Wasserstoff oder R⁷CO, R¹² und R¹³ unabhängig voneinander für Alkylreste mit 1 bis 4 Kohlenstoffatomen und X für Halogenid, Alkylsulfat oder Alkylphosphat steht. Derartige Amidesterquats sind beispielsweise unter der Bezeichnung Incroquat (Croda) im Markt erhältlich.

Hinsichtlich der Auswahl der bevorzugten Fettsäuren und des optimalen Veresterungsgrades gelten die für **(V)** genannten Beispiele auch für die Esterquats der Formeln **(VI)** und **(VIII)**. Üblicherweise gelangen die Esterquats in Form 50 bis 90 Gew.-%iger alkoholischer Lösungen in den Handel, die bei Bedarf problemlos mit Wasser verdünnt werden können. Im Sinne des erfindungsgemäßen Verfahrens können die Esterquats in Mengen von 0,1 bis 10, vorzugsweise 0,5 bis 5 und insbesondere 1 bis 3 Gew.-% - jeweils bezogen auf die keratinreduzierende Substanz bzw. das Oxidationsmittel - eingesetzt werden.

### Proteinhydrolysate

Proteinhydrolysate stellen Abbauprodukte von tierischen oder pflanzlichen Proteinen, beispielsweise Collagen, Elastin oder Keratin und vorzugsweise Mandel- und Kartoffelprotein sowie insbesondere Weizen-, Reis- und Sojaprotein dar, die durch saure, alkalische und/oder enzymatische Hydrolyse gespalten werden und danach ein durchschnittliches Molekulargewicht im Bereich von 600 bis 4000, vorzugsweise 2000 bis 3500 aufweisen. Obschon Proteinhydrolysate in Ermangelung eines hydrophoben Restes keine Tenside im klassischen Sinne darstellen, finden sie wegen ihrer dispergierenden Eigenschaften vielfach Verwendung zur Formulierung oberflächenaktiver Mittel. Übersichten zu Herstellung und Verwendung von Proteinhydrolysaten sind beispielsweise von G. Schuster und A. Domsch in **Seifen Öle Fette Wachse 108, 177 (1982)** bzw. **Cosm.Toil, 99, 63 (1984)**, von H. W. Steisslinger in **Parf.Kosm. 72, 556 (1991)** und F. Aurich et al. in **Tens.Surf.Det. 29, 389 (1992)** erschienen. Vorzugsweise werden pflanzliche Proteinhydrolysate auf Basis von Weizengluten oder Reisprotein eingesetzt, deren Herstellung in den beiden Deutschen Patentschriften **DE 19502167 C1** und **DE 19502168 C1** (Henkel) beschrieben wird. Die Proteinhydrolysate können im Sinne des erfindungsgemäßen Verfahrens auch kationisch oder anionisch modifiziert sein.

Kationische Derivate erhält man dann durch Umsetzung mit Verbindungen, die üblicherweise quartäre Ammoniumgruppen tragen oder durch Umsetzung mit entsprechenden Aminen und anschließende Quaternierung. Eine Reihe solcher quartärer Proteinhydrolysate sind als Handelsprodukte erhältlich, beispielsweise:
- kationisches Kollagenhydrolysat, beispielsweise das unter der Bezeichnung Lamequat®L auf dem Markt befindliche Produkt (INCI-Bezeichnung: Lauryldimonium Hydroxypropylamino Hydrolyzed Animal Protein; Chemische Fabrik Grünau)
- kationisches Keratinhydrolysat, beispielsweise das unter der Bezeichnung Croquat® auf dem Markt befindliche Produkt (INCI-Bezeichnung: Cocodimonium Hydroxypropyl Hydrolyzed Keratin; Croda)
- kationisches Weizenhydrolysat, erhältlich unter der Bezeichnung Hydrotriticum® QL (CTFA-Bezeichnung: Lauryldimonium Hydroxypropyl Hydrolized Wheat Protein; Croda) das unter der Bezeichnung Crotein®Q erhältliche Produkt, gemäß INCI ein "Steartrimonium Hydrolyzed Animal Protein" (Croda) sowie das als Lexein®Q X 3000 (Inolex) erhältliche quaternierte Eiweißhydrolysat.

Anionische Derivate von Proteinhydrolysaten werden üblicherweise durch Umsetzung der Proteinhydrolysate mit organischen Säuren erhalten. Solche Säuren sind beispielsweise Ölsäure, Myristinsäure, Undecylensäure, Kokosfettsäure und Abietinsäure. Die Kondensationsprodukte können auch in Form von Salzen, insbesondere Natrium-, Kalium- und Triethanolaminsalzen vorliegen. Solche Kondensationsprodukte auf Basis Kollagenhydrolysat tragen auch die INCI-Bezeichnungen Oleoyl Hydrolyzed Animal Protein, Myristoyl Hydrolyzed Animal Protein, Oleoyl Hydrolyzed Animal Collagen, Potassium Coco Hydrolyzed Animal Protein, TEA Abietoyl Hydrolyzed Animal Collagen, Potassium Undecylenoyl Hydrolyzed Animal Collagen und TEA Coco Hydrolyzed Animal Collagen. Handelsprodukte sind beispielsweise Lamepon®LPO, Lamepon®4 SK, Lamepon®UD, Lamepon®460, Lamepon®PA TR, Lamepon®ST 40 und Lamepon®S (Grünau) sowie Lexein®A 240, Lexein®S 620 und Lexein®A 520 (Inolex). Kondensationsprodukte von Elastinhydrolysaten mit Fettsäuren wie beispielsweise Laurinsäure (INCI-Bezeichnung: Lauroyl Hydrolyzed Animal Elastin) können ebenfalls eingesetzt werden. Crolastin®AS (Croda) ist ein entsprechendes Marktprodukt. Unter der Bezeichnung Promois EGCP erhältlich ist ein Potassium Cocoyl Hydrolyzed Wheat Protein; (Seiwa). Weitere erfindungsgemäß einsetzbare Handelsprodukte sind Lexein®A 200 (Inolex), Lamepon®PO-TR, Lamepon®PA-K, Lame-pon®S-MV und Lamepon®S-TR (Grünau) und Crotein®CCT (Croda).

Im Sinne des erfindungsgemäßen Verfahrens können die gegebenenfalls kationisch oder anionisch modifizierten Proteinhydrolysate in Mengen von 0,1 bis 10, vorzugsweise 0,5 bis 5 und insbesondere 1 bis 3 Gew.-% - jeweils bezogen auf die keratinreduzierende Substanz bzw. das Oxidationsmittel - eingesetzt werden.

### Gewerbliche Anwendbarkeit

Unter Einsatz von Esterquats werden Well- und Fixiermittel erhalten, die nicht nur mild sind und ausgezeichnete Eigenschaften in der Haarverformung aufweisen, sondern auch bei Temperaturlagerung nicht eindicken und eine vorteilhafte Viskosität nach Brookfield im Bereich von 4.000 bis 7.000 mPas aufweisen. Ein weiterer Gegenstand der Erfindung betrifft die Verwendung von wäßrige Zubereitung der keratinreduzierenden Substanz und/oder des Oxidationsmittels, enthaltend
(a) Zuckertenside ausgewählt aus der Gruppe, die gebildet wird von (a1) Alkyl- und/oder Alkenyloligoglykosiden und/oder (a2) Fettsäure-N-alkylpolyhydroxyalkylamiden, und
(b) Fettsäurepartialglyceride ausgewählt aus der Gruppe, die gebildet wird von Ölsäuremonoglycerid, Ölsäurediglycerid, Isostearinsäuremonoglycerid, Isostearinsäurediglycerid, Behensäuremonoglycerid, Behensäurediglycerid, Isobehensäuremonoglycerid und/oder Isobehensäurediglycerid,
zur Herstellung von Well- und Fixiermitteln, in denen sie in Mengen von jeweils 0,1 bis 20, vorzugsweise 0,5 bis 10 und insbesondere 1 bis 5 Gew.-% enthalten sein können, mit der Maßgabe, daß sich die Mengenangaben zu 100 Gew.-% ergänzen.

Im weiteren werden folgende Bezeichnungen verwendet:
- "Wellmittel" für die wäßrige Zubereitungen der keratinreduzierenden Substanz,
- "Zwischenspülung" für die erste Spülung und
- "Fixiermittel" für die wäßrige Zubereitung des Oxidationsmittels.

Weiterhin werden die Einzelheiten der erfindungsgemäßen Lehre anhand von Dauerweilmitteln geschildert. Die entsprechenden Mittel eignen sich aber in gleichem Maße und mit den gleichen Vorteilen zum Glätten von natürlich gekräuselten oder gewellten Haaren.

### Wellmittel

Wellmittel, zu deren Herstellung man die Zuckertenside und Fettsäurepartialglyceride verwenden kann und die im Sinne des erfindungsgemäßen Verfahrens Anwendung finden, enthalten zwingend die als keratinreduzierende Substanzen bekannten Mercaptane. Solche Verbindungen sind beispielsweise Thioglykolsäure, Thiomilchsäure, Thioäpfelsäure, Mercaptoethansulfonsäure sowie deren Salze und Ester, Cysteamin, Cystein, Bunte Salze und Alkalisalze der schwefligen Säure. Bevorzugt geeignet sind die Alkali- oder Ammoniumsalze der Thioglykolsäure und/oder der Thiomilchsäure sowie die freien Säuren. Diese werden in den Wellmitteln bevorzugt in Konzentrationen von 0,5 bis 1,0 Mol/kg bei einem pH-Wert von 5 bis 12, insbesondere von 7 bis 8,5, eingesetzt. Die Wellmittel können als gebrauchsfertige Mischungen formuliert werden, die vom Friseur oder Endverbraucher direkt angewendet werden können. Es hat sich in manchen Fällen aber als vorteilhaft oder notwendig erwiesen, wenn die Mittel als sogenannte 2-Komponenten-Mischungen formuliert werden, die erst vom Anwender zum gebrauchsfertigen Wellmittel vermischt werden. In diesem Fall enthält eine Formulierung das Reduktionsmittel in einem geeigneten Träger, z.B. Wasser oder einer Emulsion.

### Fixiermittel

Zwingender Bestandteil der Fixiermittel, zu deren Herstellung man die Zuckertenside und Fettsäurepartialglyceride verwenden kann und die ebenfalls Anwendung im erfindungsgemäßen Verfahren finden, sind Oxidationsmittel, z. B. Natriumbromat, Kaliumbromat, Wasserstoffperoxid, und die zur Stabilisierung wäßriger Wasserstoffperoxidzubereitungen üblichen Stabilisatoren. Der pH-Wert solcher wäßriger Wasserstoffperoxid-Zubereitungen, die üblicherweise etwa 0,5 bis 3,0 Gew.-% H₂O₂ enthalten, liegt bevorzugt bei 2 bis 4; er wird durch anorganische Säuren, bevorzugt Phosphorsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Gleichfalls geeignet sind Fixiermittel auf enzymatischer Basis (Peroxidasen), die keine oder nur geringe Mengen an Oxidationsmitteln, insbesondere H₂O₂, enthalten. Es kann bevorzugt sein, das Oxidationsmittel als 2-Komponenten-System zu formulieren. Die beiden Komponenten, von denen die eine bevorzugt eine Wasserstoffperoxidlösung oder eine wäßrige Lösung eines anderen Oxidationsmittels ist und die andere die übrigen Bestandteile enthält, werden ebenfalls erst kurz vor der Anwendung vermischt.

### Tenside

Sowohl Well- als auch Fixiermittel können weitere Tenside in untergeordneten Mengen enthalten. Als untergeordnete Mengen werden erfindungsgemäß Mengen von weniger als 70 %, insbesondere weniger 50 % an Aktivsubstanz verstanden. Als weitere Tenside kommen prinzipiell alle für Haarbehandlungsmittel, insbesondere auf dem Verformungsgebiet, bekannten Tenside in Betracht. Dies sind:
**Anionische Tenside** wie beispielsweise Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J. Falbe (ed.), "Surfactants in Consumer Products", Springer Verlag, Berlin, 1987, S. 54-124** oder **J. Falbe (ed.), "Katalysatoren, Tenside und Mineralöladditive", Thieme Verlag, Stuttgart, 1978, S. 123-217** verwiesen.

Der Fachmann wird dabei bevorzugt solche Tenside auswählen, die aufgrund ihres geringen Reizpotentials oder ihrer Quellwirkung vorteilhaft sind. In einer besonders vorteilhaften Ausführungsform der vorliegenden Erfindung enthalten aber weder Weil- noch Fixiermittel weitere Tenside außer Alkylpolyglykosiden, Fettsäure-N-alkylglucamiden, Esterquats und pflanzlichen Proteinhydrolysaten. Auch die Zwischenspülung enthält bevorzugt neben Wasser und gelösten Salzen keine weiteren Komponenten. Weiterhin kann es von Vorteil sein, wenn Wellmittel und Fixiermittel auf der gleichen Tensidbasis aufgebaut sind.

### Hilfs- und Zusatzstoffe

Weiterhin können die Wellmittel alle für diesen Zweck bekannten Inhaltsstoffe enthalten, wie z.B. Perlglanzwachse, Konsistenzgeber, Verdickungsmittel, Polymere, Siliconverbindungen, Fette, Wachse, Stabilisatoren, biogene Wirkstoffe, Antischuppenmittel, Filmbildner, Quellmittel, Hydrotrope, Strukturanten, Komplexbilder, Trübungsmittel, Treibmittel, Konservierungsmittel, Solubilisatoren, UV-Lichtschutzfilter, Insektenrepellentien, Selbstbräuner, Parfümöle, Farbstoffe und dergleichen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976**).

Typische Beispiele für **Fette** sind Glyceride, als **Wachse** kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Strukturanten** eignen sich beispielsweise Glucose oder Maleinsäure. Als **Komplexbildner** können EDTA, Phenazetin, NTA und Phosphonsäuren eingesetzt werden. Als **Trübungsmittel** dient beispielsweise Latex.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim,** **1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hufs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

Sowohl Wellmittel als auch Fixiermittel können als Creme, Gel oder Flüssigkeit formuliert sein. Weiterhin ist es möglich, die Mittel in Form von Schaumaerosolen zu konfektionieren, die mit einem verflüssigten Gas wie z. B. Propan-Butan-Gemischen, Stickstoff, Kohlendioxid, Luft, Distickstoffoxid, Dimethylether, Fluorchlorkohlenwasserstofftreibmitteln oder Gemischen davon in Aerosolbehältern mit Schaumventil abgefüllt werden. Die Well- und Fixiermittel können dabei mit allen, dem Fachmann bekannten, üblichen Vorbehandlungsmitteln, Zwischenspülungen und/oder Nachbehandlungsmitteln (zur Verbesserung von Avivage und Haltbarkeit der Frisur) kombiniert werden.

### Beispiele

Die folgenden Beispiele illustrieren die Herstellung von Fixierlösungen auf Basis der erfindungsgemäßen Tenside (**1,2, 3**) und Panthenol (**V1, V2**). Zur Herstellung der Zubereitungen wird Wasser auf 75°C erhitzt, die Zuckertenside und Partialglyceride sowie gegebenenfalls die weiteren Tenside eingerührt und homogenisiert. Anschließend läßt man die Mischung bis auf 40 °C abkühlen und rührt dann die übrigen Inhaltsstoffe ein. Darüber hinaus werden Humanhaare (Alkinco 6634) nach der Anwendung der in Tabelle 1 aufgeführten Mischungen einer thermischen Analyse unterworfen. Mit Hilfe der dynamischen Differenz-Kalorimetrie (HP-DSC; F.J. Wortmann et al., J. Appl. Polym. Sci. 1993, 48, S. 137ff.) wird der Umwandlungspunkt der behandelten Haarprobe im Vergleich zur unbehandelten Haarprobe (HP-DSC=152,5°°C) gemessen. Die Zusammensetzung der Fixiermittel ist in Tabelle 1 wiedergegeben.

**Tabelle 1**

| **Fixiermittel (Mengenangaben als Gew.-%)** | | | | |
|---|---|---|---|---|
| **Zusammensetzung/Performance** | **1** | **2** | **3** | **V1** |
| **Lamesoft® PO 65** | 3,0 | 4,0 | 5,0 | - |
| Coco Glucosid, (and) Glyceryl Oleate | | | | |
| **Dehyquart® C 4046** | - | 1,0 | - | - |
| Cetaryl Alkohol (and) Dipalmitoylethyl Hydroxyethylmonium Methosulfate (and) Cetearyl Sulfate | | | | |
| Panthenol | - | - | - | 3,0 |
| **Guadin® WQ** | - | - | 1,2 | 1,2 |
| Lauridominium Hydroypropyl Hydrolyzed Wheat Protein | | | | |
| **Turpinal ® SL** | 0,3 | 0,3 | 0,3 | 0,3 |
| Etidronic Acid | | | | |
| Wasserstoffperoxid (35 Gew.-%ig) | 7,5 | 7,5 | 7,5 | 7,5 |
| Wasser | ad 100 | | | |
| **pH-Wert** | 2,5 | 2,7 | 2,8 | 2,8 |
| **Brookfield-Viskosität, [mPas] (23 °C, Sp. TC, 10 Upm)** | 4.900 | 3.600 | 6.150 | 6.150 |
| **Umwandlungspunkt nach HP-DSC** | 140,4 | 150,7 | 151,1 | 149,8 |

## Patentansprüche

1. Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült, **dadurch gekennzeichnet,** daß die wäßrige Zubereitung der keratinreduzierenden Substanz und/oder des Oxidationsmittels
(a) 0,1 bis 20 Gew.-% Zuckertenside ausgewählt aus der Gruppe, die gebildet wird von (a1) Alkyl- und/oder Alkenyloligoglykosiden und/oder (a2) Fettsäure-N-alkylpoly-hydroxyalkylamiden und
(b) 0,1 bis 20 Gew.-% Fettsäurepartialglyceride
enthält, mit der Maßgabe, daß sich die Mengenangaben mit Wasser sowie gegebenenfalls weiteren Hilfs- und Zusatzstoffen zu 100 Gew.-% ergänzen.

2. Verfahren nach Anspruch 1, **dadurch gekennzeichnet**, daß sie als Komponente (a1) Alkyl- und Alkenyloligoglykoside der Formel **(I)** einsetzt, 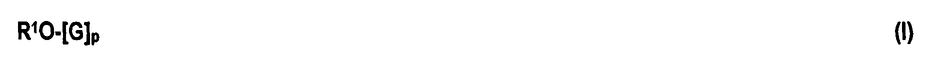 in der R¹ für einen Alkyl- und/oder Alkenylrest mii 4 bis 22 Kohlenstoffatomen, G für einen Zuckerrest mit 5 oder 6 Kohlenstoffatomen und p für Zahlen von 1 bis 10 steht.

3. Verfahren nach den Ansprüchen 1 und/oder 2, **dadurch gekennzeichnet**, daß sie als Komponente (a2) Fettsäure-N-alkylpolyhydroxyalkylamide der Formel **(II)** einsetzt, 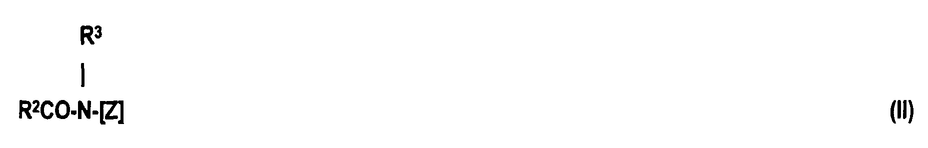 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

4. Verfahren nach mindestens einem der Ansprüche 1 bis 3, **dadurch gekennzeichnet**, daß sie als Komponente (b) Fettsäurepartialglyceride der Formel **(IV)** einsetzt, 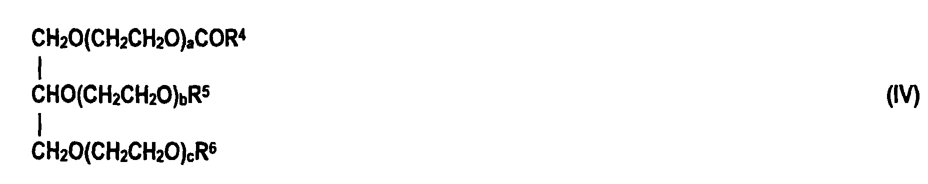 in der R⁴CO für einen linearen oder verzweigten, gesättigten und/oder ungesättigten Acylrest mit 6 bis 22, vorzugsweise 12 bis 18 Kohlenstoffatomen, R⁵ und R⁶ unabhängig voneinander für R⁴CO oder OH und die Summe (a+b+c) für 0 oder Zahlen von 1 bis 100, vorzugsweise 5 bis 25 steht, mit der Maßgabe, daß mindestens einer der beiden Reste R⁵ und R⁶ OH bedeutet.

5. Verfahren nach mindestens einem der Ansprüche 1 bis 4, **dadurch gekennzeichnet**, daß man als weitere Tenside Esterquats der Formel **(V)** einsetzt, 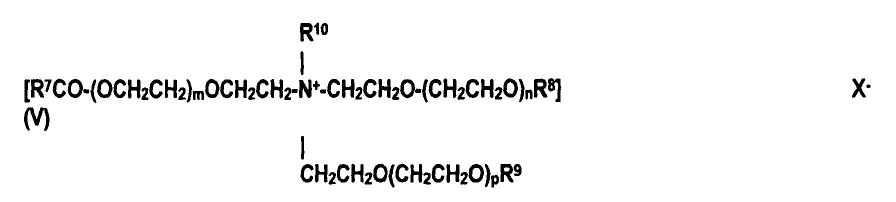 in der R⁷CO für einen Acylrest mit 6 bis 22 Kohlenstoffatomen, R⁸ und R⁹ unabhängig voneinander für Wasserstoff oder R⁷CO, R¹⁰ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

6. Verfahren nach mindestens einem der Ansprüche 1 bis 5, **dadurch gekennzeichnet**, daß man als weitere Tenside gegebenenfalls kationisch oder anionisch modifizierte Proteinhydrolysate einsetzt.

7. Verfahren nach mindestens einem der Ansprüche 1 bis 6, **dadurch gekennzeichnet**, daß man die weiteren Tenside in Mengen von jeweils 0,1 bis 10 Gew.-% - bezogen auf die keratinreduzierende Substanz bzw. das Oxidationsmittel - einsetzt.

8. Verwendung von wäßrigen Zubereitungen der keratinreduzierenden Substanz und/oder des Oxidationsmittels, enthaltend
(a) 0,1 bis 20 Gew.-% Zuckertenside ausgewählt aus der Gruppe, die gebildet wird von (a1) Alkyl- und/oder Alkenyloligoglykosiden und/oder (a2) Fettsäure-N-alkylpoty-hydroxyalkylamiden und
(b) 0,1 bis 20 Gew.-% Fettsäurepartialglyceride
mit der Maßgabe, daß sich die Mengenangaben mit Wasser sowie gegebenenfalls weiteren Hilfs- und Zusatzstoffen zu 100 Gew.-% ergänzen, zur Herstellung von Well- und Fixiermitteln.
